# EUROPEAN PATENT SPECIFICATION

(11) **EP 1 352 664 B1**
(45) Date of publication and mention of the grant of the patent: **22.09.2004**
(21) Application number: 01984290.5
(22) Date of filing: 19.07.2001
(51) Int. Cl.: A61L 9/014, A61L 9/16, B01J 20/04, B01J 20/12, B01J 20/30, B01D 53/44

(54) **DEODORANT MATERIAL AND METHOD FOR PREPARATION THEREOF**
DESODORIERENDES MITTEL UND VERFAHREN ZU DESSEN HERSTELLUNG
MATIERE DESODORISANTE ET PROCEDE DE PREPARATION ASSOCIE

(30) Priority: 21.07.2000 JP 2000220351; 18.01.2001 JP 2001010758
(43) Date of publication of application: 15.10.2003
(73) Proprietor: Midori Anzen Co., Ltd., Tokyo 150-8455 (JP)
(72) Inventor: ICHINOSE, Hidehito, c/o MIDORI ANZEN CO. LTD., Tokyo 150-8455 (JP); KOSHIO, Hisashi, c/o MIDORI ANZEN CO. LTD., Tokyo 150-8455 (JP)
(74) Representative: Strehl Schübel-Hopf & Partner
(86) International application number: PCT/JP2001/006264
(87) International publication number: WO 2002/007790

(56) References cited:
- WO-A-82/04408
- WO-A-92/16291
- JP-A- 2 095 376
- JP-A- 2 098 360
- JP-A- 58 073 364
- JP-A- 61 136 438
- JP-A- 63 108 083
- JP-A- 63 161 968
- JP-A- 2000 152 728
- PATENT ABSTRACTS OF JAPAN vol. 016, no. 485 (C-0993), 8 October 1992 (1992-10-08) -& JP 04 176466 A (HOKKAIDO ELECTRIC POWER CO INC:THE), 24 June 1992 (1992-06-24) -& DATABASE WPI Derwent Publications Ltd., London, GB; AN 1992-262128 XP002247047 & JP 04 176466 A ( HOKKAIDO ELECTRIC POWER CO INC)
- PATENT ABSTRACTS OF JAPAN vol. 010, no. 328 (C-383), 7 November 1986 (1986-11-07) -& JP 61 136438 A (TOYOTA CENTRAL RES & DEV LAB INC), 24 June 1986 (1986-06-24) -& DATABASE WPI Derwent Publications Ltd., London, GB; AN 1986-202360 XP002247048 & JP 61 136438 A (TOYOTA CENT RES & DEV LAB)
- PATENT ABSTRACTS OF JAPAN vol. 009, no. 059 (C-270), 15 March 1985 (1985-03-15) -& JP 59 193979 A (ENCLER BUSINESS KK), 2 November 1984 (1984-11-02) -& DATABASE WPI Derwent Publications Ltd., London, GB; AN 1984-309786 XP002247050 & JP 59 193979 A (ENCLER BUSINESS KK)

## Description

### Technical Field

The present invention relates to a deodorizing article which can be used semi-permanently, and more particularly to a deodorizing article which effectively removes an oil-related odor generated from a kitchen. The invention also relates to a method for producing the deodorizing article.

### Background Art

Conventionally, there have been known methods for removing offensive odor substances from a variety of sources of offensive odor; e.g., a deodorization method by adsorption ; a chemical deodorization method including neutralization and decomposition of an odorous substance by use of a chemical; and a biological deodorization method including decomposition of an odor component by use of a microorganism.

Generally, the adsorption deodorization method employs activated carbon. After the activated carbon has been used for a certain period of time, the effect of activated carbon weakens considerably to such a level that substantially no deodorization is expected. Therefore, the deodorizing material must be replaced by new material at predetermined intervals, which is cumbersome.

A problem arises upon employment of the chemical deodorization method. Specifically, the method requires direct contact between an odorous substance and a chemical for causing chemical reaction. Thus, such a chemical requires additional care such as periodical replacement or replenishment, and handling of chemicals involves hazard. This situation is also problematic.

The biological deodorization method is less hazardous. However, the method also involves problems such as requiring a large deodorization facility and a water-supply facility.

These conventional methods require maintenance work such as replacement/replenishment of material or supply of water. In the event of failure to perform the maintenance, the article and method employed for deodorization fail to function. In other words, an offensive odor cannot be removed even though the deodorizing article is employed in combination with the deodorizing method.

Examples of conventional odor adsorbing materials and filters can be found in prior art publications WO-A-92/16291, JP-A-04176466, and JP-A-61136438.

In view of the foregoing, an object of the present invention is to provide a deodorizing article which can be used semi-permanently. Another object of the invention is to provide a method for producing the deodorizing article.

The target of conventional deodorization methods is 100% removal of an odor; i.e., complete deodorization. However, the present inventors have realized that a maintenance-free deodorizing article which can be used semi-permanently serves as a sufficiently effective deodorizing article, from the whole aspect, so long as the deodorizing article attains a certain level of deodorization. The present invention has been accomplished on the basis of this finding.

More specifically, in the city of Tokyo, the odor concentration of exhaust from kitchens of restaurants and other food businesses is restricted to fall under a regulation standard in accordance with the Tokyo Metropolitan Environmental Pollution Ordinance. Under these circumstances, althrough pursuing a deodorizing article which attains complete deodorization may have some meaning, from a viewpoint that takes into consideration use of the article involving the aforementioned maintenance, a deodorizing article which can suppress the odor concentration to a level below regulation standard determined by, for example, ordinances or to a level below a certain concentration (e.g., a low odor concentration where an offensive odor is not sensed) and which can be used semi-permanently could be more effective. The present inventors have considered this viewpoint.

### Disclosure of the Invention

In order to solve the aforementioned problems the present invention provides a deodorizing article according to claim 1. The subclaims relate to preferred modifications.

The meso-pores of the clay mineral involve repeated adsorption and desorption of an odor substance, in cooperation with the moisture-maintenance effect of the gypsum series. Thus, the deodorizing article for use in an exhaust system where an odor component is present exerts a semi-permanent deodorization effect. Sepiolite or palygorskite exerts a semi-permanent deodorization effect on the basis of adsorption and desorption of an odor substance, in cooperation with the moisture-maintenance effect of the gypsum series. The amount of gypsum series is at least 25 wt.%, such that the shape of the deodorizing article is satisfactorily maintained.

By the modification of claim 2, clogging or similar trouble of the deodorizing article caused by an odor component which has been adsorbed by the deodorizing article and not yet released from the deodorizing article, i.e. an odor component which has been accumulated in the deodorizing article, can be overcome through enzymatic reaction involved with a microorganism adhering thereon, to thereby renew the adsorption effect.

By the modification of claim 3, an offensive odor such as an oil-related odor generated in a kitchen is reduced to a certain level or lower by causing exhaust of the kitchen to flow through the flow line of the deodorizing article placed in an exhaust system.

According to the modification of claim 4, the deodorizing article further comprises an additional mineral between the gypsum series and the clay mineral. Thereby, the desorption of substances which have been adsorbed by the clay mineral is promoted.

By using expanded perlite as the additional mineral (claim 5), the desorption of substances which have been adsorbed by the clay mineral is effectively promoted by the presence of macro-pores in the expanded perlite.

If the deodorizing article contains the additional mineral in the preferred amount of 30 wt.% or less (claim 6), the form of the deodorizing article is satisfactorily maintained, and there can be attained an excellent balance between adsorption and desorption.

Claims 7 to 10 relate to corresponding methods.

The present invention will next be described in detail.

The clay mineral having a tunnel-like micro-structure which mineral can be used in the present invention is sepiolite, palygorskite, or a similar mineral. These minerals generally have one-dimensional tunnel pores (i.e., micro-pores) and meso-pores. For example, sepiolite has micro-pores of 0.5 to 1.1 nm originating from the crystal structure thereof, and meso-pores of some tens of nm corresponding to interparticle spacing.

Such minerals are generally known to exert moisture-controlling effect. According to the present invention, a semi-permanent deodorization effect on the basis of adsorption and desorption of an odor substance is exerted in cooperation with the moisture-maintenance effect of the gypsum series.

No particular limitation is imposed on the particle size of the clay mineral. The particle is generally about 0.2 to about 2 mm.

The gypsum series which can be employed in the present invention is a compound which comprises calcium sulfate as a predominant component and can be molded into a desired shape through reaction with water. Either naturally occurring gypsums or chemically synthesized gypsums may be employed. Examples of the gypsums include crystalline gypsum, hemihydrate, anhydrous gypsum, and plaster of Paris. Generally, commercial plaster of Paris (calcined gypsum) is used.

The clay mineral having a tunnel-like micro-structure of the present invention is used in an amount of at least 30 wt.% in order to attain considerable adsorption performance.

The gypsum series must be used in an amount of at least 25 wt.% for maintaining the shape of the deodorizing article. Thus, when the deodorizing article is formed from two components, the gypsum series content is preferably 25 to 70 wt.%.

The aforementioned gypsum series maintains the shape of the deodorizing article of the present invention and serves as a water-supply material. Thus, the gypsum series comprises a material which provides macro-pores of 1 to 10 µm, preferably about 5 µm, after molding thereof.

The deodorizing article of the present invention contains gypsum which surrounds sepiolite having micro-pores and meso-pores. The gypsum, having macro-pores of 1 to 10 µm, serves as a water-supply material. An adsorption-desorption mechanism involving sepiolite and gypsum is considered as follows. When an atmosphere or air which passes through the deodorizing article contains an odor component at high level, sepiolite adsorbs the odor component which has passed through pores of gypsum, and in turn transfers water which has been adsorbed by sepiolite to gypsum, to thereby lower the odor level to a certain level or lower.

Even when the odor component does not exist, gypsum continues to adsorb water until adsorption of water reaches the saturation state. Sepiolite, which exerts moisture-controlling effect, adsorbs water contained therein. The odor component which has been adsorbed by sepiolite is gradually released through substitution; i.e., competitive adsorption, to thereby lower the odor level to a certain level or lower (i.e., desorption).

On the basis of the function of water, the deodorizing article of the present invention exerts particularly remarkable effect of removing an oil-related odor which is generated in a place such as a kitchen where vaporization of water is prone to occur during the course of cooking.

As described above, by virtue of the synergistic effect exerted by gypsum series and a clay mineral such as sepiolite, the deodorizing article of the present invention adsorbs an odor substance to thereby lower the odor level to a certain level or lower, when the target gas contains a large amount of an odor component, and releases the odor component without elevating the level of the released odor component to a certain level or higher, when the odor component level is lowered. By repeating the procedure, the odor level can be averaged and lowered to a certain level or lower, with the level being suppressed semi-permanently.

The deodorizing article of the present invention preferably contains a mineral having a pore size greater than that of gypsum series, in view of promotion of the aforementioned desorption of the odor substance.

Examples of minerals having a pore size greater than that of gypsum series include expanded perlite (may be referred to as foamed perlite). The expanded perlite, which per se has no adsorption ability, increases the entire volume of a mixture of gypsum series and a clay mineral having a tunnel-like micro-structure when incorporated into the mixture. Since the macro-pore size of the additional mineral is greater than that of gypsum series, the aforementioned adsorption-desorption is considered to be promoted.

The amount of the mineral having a large pore size is controlled so as not lower the relative amounts of active ingredients and so as not to greatly lower overall mechanical strength. Thus, the amount is controlled to 30 wt.% or less, preferably 8 to 12.5 wt.%. Although the additional mineral is optional, the mineral is preferably added in an amount of at least 8 wt.% in order to fully attain the effect thereof.

No particular limitation is imposed on the particle size of expanded perlite, and the particle size is generally 0.15 to 1 mm.

The deodorizing article of the present invention is produced by mixing raw materials with dispersion, charging the resultant mixture into a mold, and shaping the mixture. No particular limitation is imposed on the shape, dimensions, etc. of the deodorizing article. However, since the deodorization is effected on the basis of adsorption (contact with the odor substance), the deodorizing article preferably has a structure assuring a large contact area. For example, a columnar article or a prismatic article of a honeycomb structure having a flow line penetrated in an axial direction is preferred. A typical hollow cylinder, prism, etc. may also be employed.

No particular limitation is imposed on the method for producing the deodorizing article of the present invention. After completion of mixing and dispersing raw materials, molding the resultant mixture by drying can be performed under the same conditions as employed for yielding typical gypsum series products.

However, as mentioned in the Test Examples below, preliminary firing of sepiolite is preferably performed before mixing and dispersing of raw materials. Preliminary firing is considered to prevent crushing or similar trouble of sepiolite during mixing and dispersion of raw materials, thereby enhancing desorption performance of the molded deodorizing article. No particular limitation is imposed on the firing conditions, so long as the aforementioned effect is attained. For example, firing is performed at 400 to 800°C for about one hour.

According to the deodorizing article of the present invention, the clay mineral contained therein and having a tunnel-like micro-structure exerts a semi-permanent deodorization effect on the basis of repeated adsorption and desorption of an odor substance, in cooperation with a moisture-maintenance effect of the gypsum series.

When a microorganism is caused to adhere to the deodorizing article (the microorganism being not intentionally caused to adhere during use of the deodorizing article of the present invention), the microorganism ingests, as nutrient, an odor component adsorbed by the deodorizing article. In addition, the microorganism produces an enzyme under the conditions satisfying requirements for water content, temperature, etc.

By virtue of enzymatic reaction, an accumulated odor component which has been adsorbed but not desorbed is decomposed, to thereby prevent clogging or similar trouble and renew adsorption effect, leading to further semi-permanent deodorization performance. Such performance regeneration effect due to a microorganism is attributed to a microorganism present in the atmosphere. Thus, the effect is generally exerted when the deodorizing article is used in the atmosphere. Particularly when the article is used in a kitchen for removing an oil-related odor, an oil-decomposing enzyme such as lipase or lipase-producing bacteria *per se* may be caused to adhere to the article in advance.

### Brief Description of the Drawings

Fig. 1 is a schematic perspective view of the deodorizing article according to one embodiment of the present invention.
Fig. 2 shows the procedure of Test Example 1 carried out in relation to the present invention.
Fig. 3 is a graph showing the results of Test Example 1 carried out in relation to the present invention.
Fig. 4 is a graph showing the results of Test Example 2 carried out in relation to the present invention.
Fig. 5 is a graph showing the results of Test Example 3 carried out in relation to the present invention.
Fig. 6 shows the procedure of Test Example 5 carried out in relation to the present invention.
Fig. 7 is a graph showing the results of Test Example 5 carried out in relation to the present invention.
Fig. 8 is a graph showing the results of Test Example 6 carried out in relation to the present invention.
Fig. 9 is a graph showing the results of Test Example 7 carried out in relation to the present invention.
Fig. 10 is a graph showing the results of Test Example 8 carried out in relation to the present invention.
Fig. 11 shows the procedure of Test Example 9 carried out in relation to the present invention.

### Best Mode for Carrying Out the Invention

The present invention will next be described in detail by way of examples.

### <Example 1>

Sepiolite (particle size: 0.5 to 1.0 mm, naturally occurring in Turkey) was subjected to preliminary firing at 600°C for one hour. The thus-fired sepiolite and grade-B calcined gypsum (1 : 1 by weight) were mixed together, and an equiamount of water was added to the mixture. The mixed product was charged in a mold of a predetermined shape, and dried at 60°C for 12 hours, to thereby yield plates 11 (thickness: 10 mm, width: 100 mm, length: 200 mm). Four plates 11 were adjoined, to thereby form a deodorizing article 10 in the form of a hollow quadrangular prism.

### <Example 2>

The procedure of Example 1 was repeated, except that the raw materials employed in Example 1 were replaced by preliminary fired sepiolite, grade-B calcined gypsum, and expanded perlite (Trade name: Topco Perlite) (proportions (wt.): 5 : 4 : 1), to thereby produce a deodorizing article.

### <Comparative Example 1>

The procedure of Example 1 was repeated, except that sepiolite was replaced by activated carbon (derived from coconut) (particle size: 0.5 to 1.0 mm), to thereby produce an adsorbing article of Comparative Example 1.

### <Comparative Example 2>

The procedure of Example 2 was repeated, except that sepiolite was replaced by activated carbon (derived from coconut) (particle size: 0.5 to 1.0 mm), to thereby produce an adsorbing article of Comparative Example 2.

### <Test Example 1>

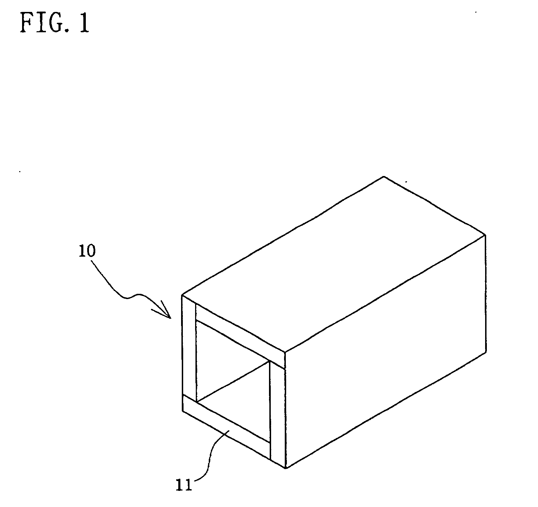

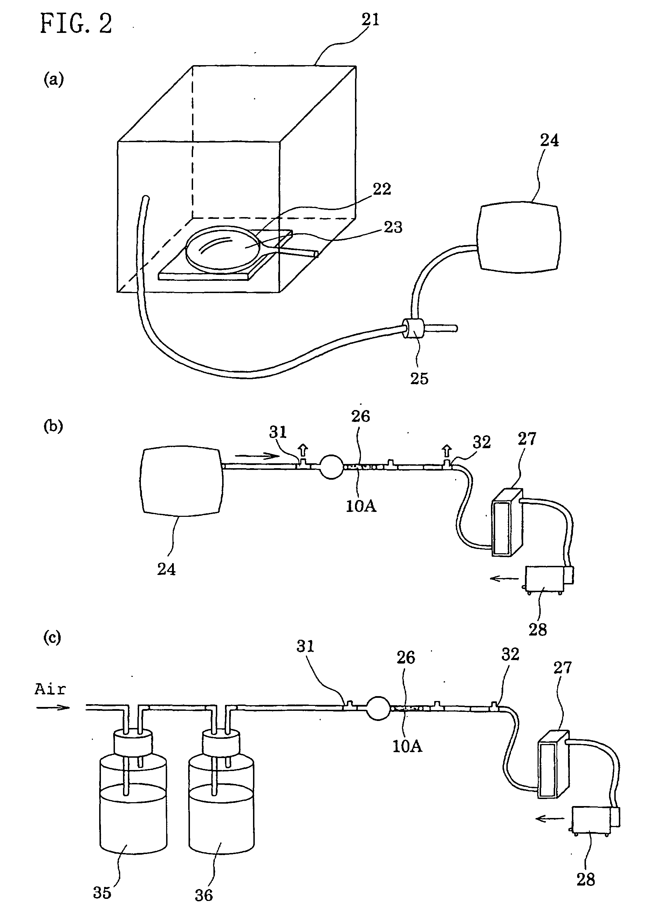

Plates which had been produced in each of Examples and Comparative Examples were pulverized, to thereby provide test samples. Each sample was subjected to an oil-related gas adsorption and desorption repeating test in the following manner. Fig. 2 shows the procedure of the test.
(1) A sample obtained from the deodorizing article (see FIG. 1) was dried in an atmosphere at about 50°C for 24 hours (preliminary treatment), after which the dried sample was allowed to stand in a 50%-RH atmosphere at about 20°C for 24 hours.
(2) Oil 23 was added to a frying pan 22 placed in a chamber 21 and heated so as to generate oil-related gas. The gas present in the chamber 21 was sucked into a 20-L sampling bag 24 (hereinafter referred to simply as "bag") by means of a pump 25 (Fig. 2(a)).
(3) The gas concentration contained in the bag 24 was controlled to 20 to 25 ppm (CH₄).
(4) A test sample 10A (2 g) was charged into a sample tube 26. One end of the sample tube was connected to the bag 24, and the other end of the sample tube was connected to a pump 28 via a flow meter 27.
(5) By operating the pump 28, the oil-related gas contained in the bag 24 was fed at 0.5 L/min into the sample tube 26. The gas was sampled every one minute for 31 minutes at an inlet sampling position 31 (upstream with respect to the sampling tube 26) and an outlet sampling position 32 (downstream with respect to the sampling tube 26) (Fig. 2(b)).
(6) Each gas sample was analyzed by means of a total hydrocarbon meter (detection limit: 0.1 ppm CH₄), and the amount of adsorbed gas component was calculated by integrating the difference in gas level between the inlet sampling potion and the outlet sampling position.
(7) The bag 24 was removed from the sample tube 26, and a silica gel trap 35 was connected to the sample tube 26 via an activated carbon trap 36. Air which had been passed through silica gel and activated carbon was fed to the sample tube 26 at 0.5 L/min for 15 minutes, to thereby effect desorption of the adsorbed gas component from the test sample charged in the sample tube 26 (Fig. 2(c)).
(8) The sample tube 26 was removed and allowed to stand at 32°C for two days.
(9) The procedure including steps (2) to (8) was repeated.

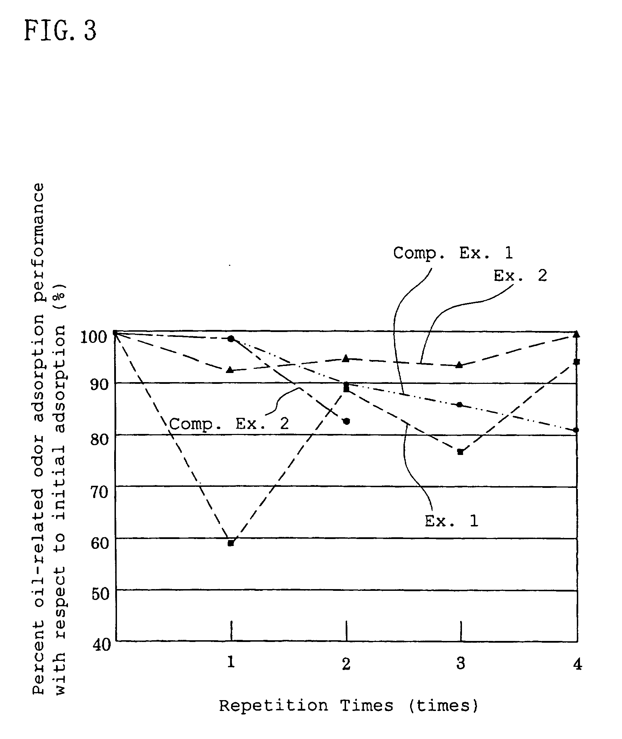

The results, shown in Fig. 3, indicate that the deodorizing article of Example 1 and that of Example 2 attain sufficient renewal of oil-related odor adsorption capacity after repeated desorption processes. At the fourth repetition, the capacity is renewed to at least 90% the initial capacity. In contrast, the adsorption capacity of the article of Comparative Example 1 employing activated carbon decreases in the course of operation, indicating that the adsorbed odor cannot be released. Since the article of Comparative Example 2 exhibits a performance inferior to that of Comparative Example 1 at the second repetition, subsequent testing was not performed.

### <Test Example 2>

Pulverized plates which had been produced in Example 1 were employed as test samples. A test sample which had been subjected to a procedure similar to that of Test Example 1 is denoted by "Ex. 1 sample." A test sample which had been subjected to a procedure similar to that of Test Example 1, except that a membrane filter for removing microorganisms was provided on the upstream side of the test sample during desorption step (7), is denoted by "Ex. 1a sample." A test sample which had been subjected to a procedure similar to that of Test Example 1, except that the desorption step (7) was omitted, is denoted by "Ex. 1b sample."

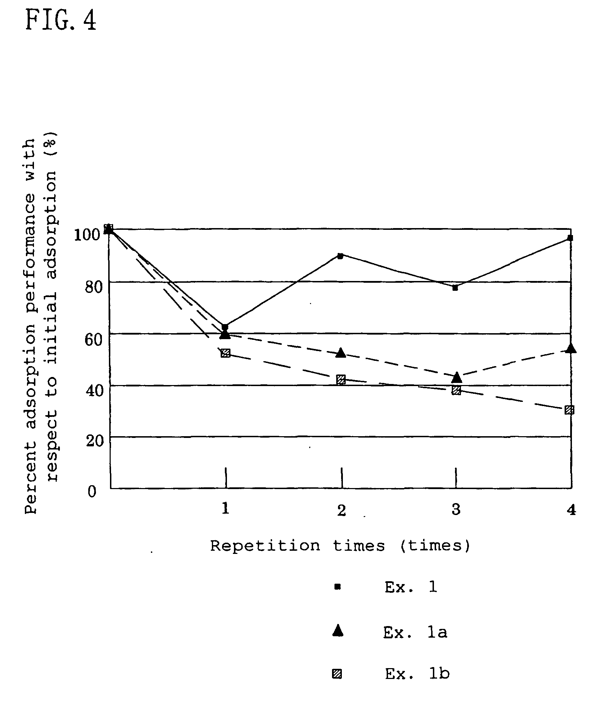

The results are shown in Fig. 4. The results indicate that as compared with Ex. 1 sample, the adsorption effect of the deodorizing article (Ex. 1a sample) decreases as steps (1) to (8) are performed repeatedly. The decrease in adsorption effect may be attributed to failure to attain microbial effect. Ex. 1b sample which had not been subjected to any desorption step exhibited further decreased adsorption effect. Thus, these results confirm that the deodorizing article of the present invention releases an adsorbed odor component, and that the adsorption effect is renewed and maintained for a long period of time by virtue of microbial effect.

### <Test Example 3>

Pulverized plates which had been produced in Example 2 were employed as test samples. A test sample which had been subjected to a procedure similar to that of Test Example 1 is denoted by Ex. 2 sample. A test sample which had been subjected to a procedure similar to that of Test Example 1, except that a membrane filter for removing microorganisms was provided on the upstream side of the test sample during desorption step (7), was denoted by Ex. 2a sample. A test sample which had been subjected to a procedure similar to that of Test Example 1, except that the desorption step (7) was omitted, was denoted by Ex. 2b sample.

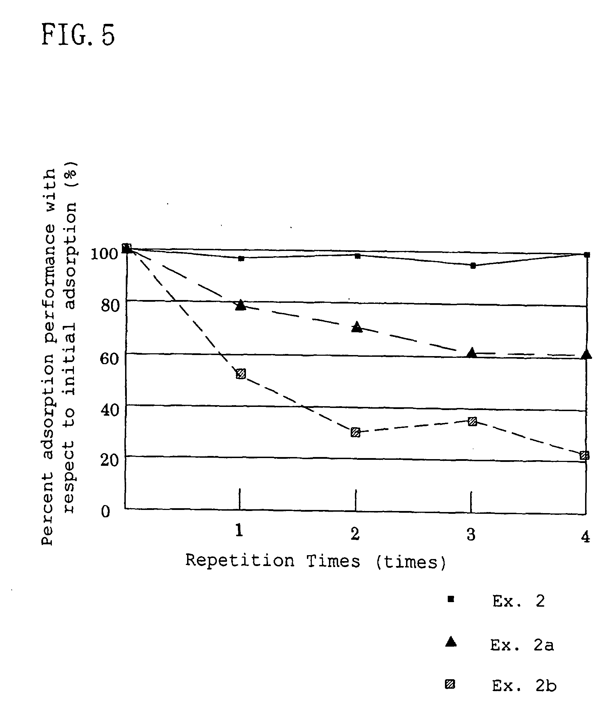

The results are shown in Fig. 5. The results indicate that the adsorption effect of the deodorizing article (Ex. 2a sample) decreases as the number of repetitions of the procedure increases, as compared with Ex. 2 sample. The decrease in adsorption effect may be attributed to failure to attain microbial effect. Ex. 2b sample which was not subjected to desorption step exhibits further decreased adsorption effect. Thus, these results confirm that the deodorizing article of the present invention releases an adsorbed odor component, and that the adsorption effect is revived and maintained in for a long period of time by virtue of microbial effect. It is also confirmed that adsorption effect of Sample Ex. 2 containing perlite is further maintained by virtue of microbial effect and in the course of repetition of desorption, as compared with Ex. 1 sample. In addition, as is clear from Fig. 3, Sample Ex. 2 containing perlite exhibited little decrease in adsorption effect when the number of repetitions of desorption is small (particularly after the first repetition), as compared with Ex. 1 sample containing no perlite.

### <Test Example 4>

The presence of bacteria exhibiting lipase activity in Ex. 2 sample which had undergone Test Example 1 was confirmed in the following manner.

Bacterial were extracted from the sample by use of a physiological saline or phosphate buffer. The bacteria were picked up and cultured isolatedly in a medium, so that a bacterial liquid was prepared. Then the bacterial liquid was divided into two portions. After completion of culturing at 32°C for two days, one portion was subjected to counting and identification of bacteria, and the other portion was subjected to determination of lipase activity (see Chemistry and Industry (Osaka Koken Kyokai) Vol. 71 (1977), p. 93 to p. 95, "Studies on Processing of Animal and Vegetable Fats and Oils" by use of Microorganisms (thesis authored by Akio SUGIHARA, Yasunori MATSUBA, Yuji SHIMADA, Yoshio TOMINAGA et al.); and Oils and Fats (Sachi Shobo), Vol. 41 (1988), p. 64 to p. 72, "Basic and Application of Enzyme Lipase (serial articles authored by Emiko IWAI)).

From the results, the bacteria isolated from the sample was identified as *Bacillus subtilis*. The lipase activity provided by the bacteria was found to be 0.19 (unit/ml·colony), which is 1.9 times the lipase activity (0.099 (unit/ml·colony)) of the bacteria isolated from a microorganism preparation (Trade name ; Oil-Gutter, product of Daishu Corporation)

### <Test Example 5>

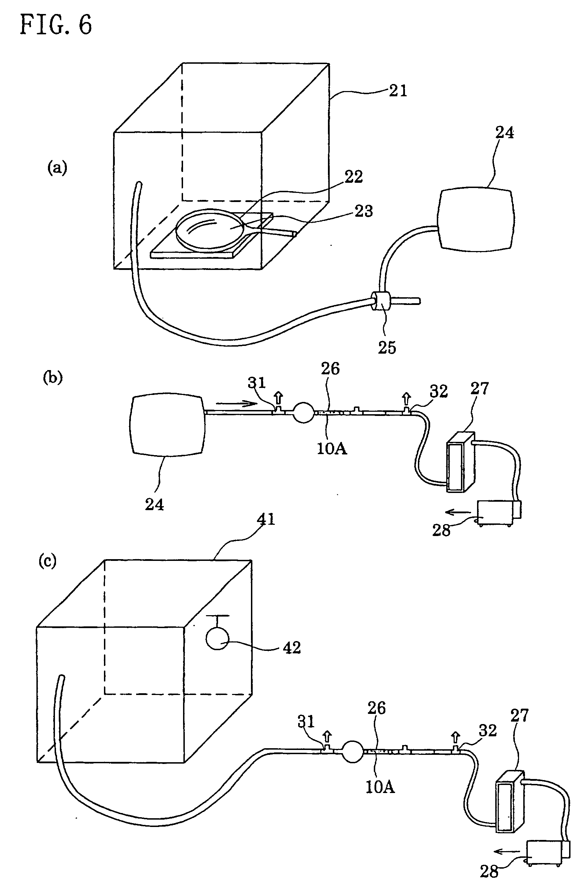

Plates which had been produced in each of Examples 1 and 2 and Comparative Example 1 were pulverized, to thereby provide test samples. Each sample was subjected to an oil-related gas adsorption and desorption repeating test in the following manner. Fig. 6 illustrates the procedure of the test.
(1) A sample obtained from the deodorizing article (see FIG. 1) was dried in an atmosphere at about 50°C for 24 hours (preliminary treatment), and subsequently the dried sample was allowed to stand in a 50%-RH atmosphere at about 20°C for 24 hours.
(2) Oil 23 was added to a frying pan 22 placed in a chamber 21, and heated so as to generate oil-related gas. The gas present in the chamber 21 was sucked into a 20-L sampling bag 24 (hereinafter referred to simply as "bag") by means of a pump 25 (Fig. 6(a)).
(3) The gas concentration contained in the bag 24 was controlled to 20 to 25 ppm (CH₄).
(4) A test sample 10A (2 g) was charged into a sample tube 26. One end of the sample tube was connected to the bag 24, and the other end of the sample tube was connected to a pump 28 via a flow meter 27.
(5) By operating the pump 28, the oil-related gas contained in the bag 24 was fed at 0.5 L/min into the sample tube 26. The gas was sampled every one minute for 31 minutes at an inlet sampling position 31 (upstream with respect to the sampling tube 26) and an outlet sampling position 32 (downstream with respect to the sampling tube 26) (Fig. 6(b)).
(6) Each gas sample was analyzed by means of a total hydrocarbon meter, and the amount of adsorbed gas component was calculated by integrating the difference in gas level between the inlet sampling potion and the outlet sampling position.
(7) The bag 24 was removed from the sample tube 26, and a chamber 41 containing moisture-controlled air and having a thermo-hygrometer 42 was connected to the sample tube 26. Air contained in the chamber 41 was fed to the sample tube at 0.5 L/min, and the gas was sampled at an inlet sampling position 31 and an outlet sampling position 32 until the difference in the gas concentration between the inlet sampling position and the outlet sampling position reached zero (i.e., until the released gas was not detected). The amount of released gas component was calculated by integrating the difference in the gas concentration between the inlet sampling position and the outlet sampling position (Fig. 6(c)).
(8) The desorption test was performed twice under different sets of temperature and moisture conditions in the chamber 41; i.e., 21 to 23°C and 46 to 55% RH, and 21 to 23°C and 75 to 79% RH.

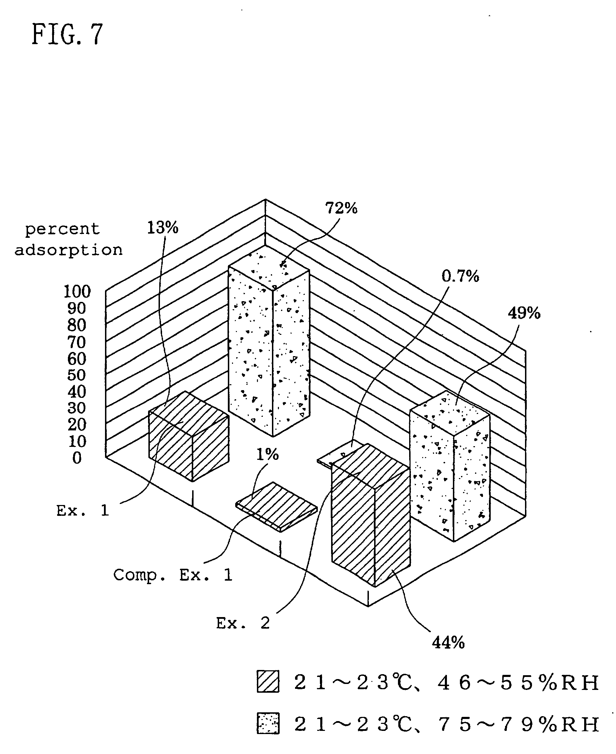

The results, which are shown in Fig. 7, indicate that the deodorizing article of Example 1 and that of Example 2 exhibit a remarkably large gas desorption amount, as compared with the deodorizing article of Comparative Example 1. The deodorizing article of Example 1 containing no perlite exhibited large percent desorption only under high-humidity conditions. However, the deodorizing article of Example 2 containing perlite exhibited average percent desorption under all humidity conditions employed in the test.

### <Example 3>

The procedure for Example 2 was repeated, except that the compositional proportions by weight of sepiolite, gypsum, and perlite was changed to 4 : 3 : 1, to thereby produce a deodorizing article.

### <Example 4>

The procedure for Example 2 was repeated, except that the compositional proportions by weight of sepiolite, gypsum, and perlite was changed to 6 : 5 : 1, to thereby produce a deodorizing article.

### <Example 5>

The procedure for Example 2 was repeated, except that the compositional proportions by weight of sepiolite, gypsum, and perlite was changed to 3 : 2 : 1, to thereby produce a deodorizing article.

### <Example 6>

The procedure for Example 2 was repeated, except that the compositional proportions by weight of sepiolite, gypsum, and perlite was changed to 2 : 1 : 1, to thereby produce a deodorizing article.

### <Test Example 6>

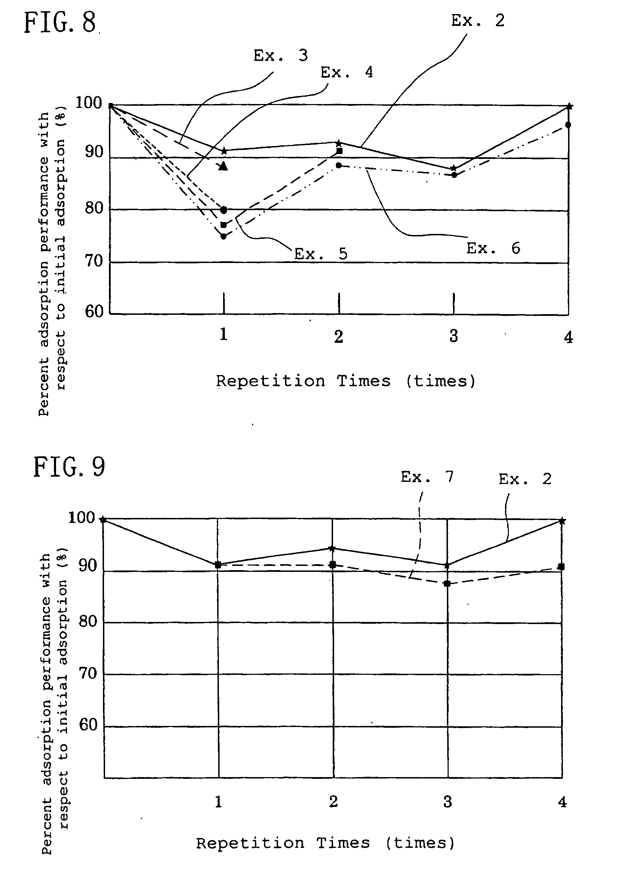

The deodorizing articles of Examples 2 to 6 were tested in a manner similar to that of Test Example 1 so as to compare the initial performance and the performance after repeated use. Fig. 8 shows the results.

The results confirm that the deodorizing articles containing perlite about 8 wt.% (Examples 4) to about 25 wt.% (Examples 6), respectively, exhibit higher performance, particularly after the first repetition, as compared with the deodorizing article of Example 1 containing no perlite as shown in Fig. 3.

Since the deodorizing articles of Examples 3 to 5 exhibit a performance superior to that of Example 6, the second or subsequent testing was not performed.

### <Example 7>

The procedure of Example 2 was repeated, except that conditions of preliminary firing of the employed sepiolite were altered to 200°C for one hour, to thereby produce a deodorizing article.

### <Test Example 7>

The deodorizing articles of Examples 2 and 7 were tested in a manner similar to that of Test Example 1 so as to compare the initial performance and the performance after repeated use. Fig. 9 shows the results.

The results indicate that conditions of preliminary firing of sepiolite affect desorption performance. When the surface of the deodorizing article produced in Example 2 was observed under a microscope, pores were observed in the surface thereof. Portions of the pores observed in the surface of the deodorizing article of Example 7 were observed as if they were covered with a molten substance. The surface conditions are considered to be provided by a portion of sepiolite particles are micro-pulverized during the mixing-dispersing step, and the resultant micro-powder bury the pores and are solidified therein.

Thus, sepiolite is subjected to preliminary firing conceivably under the conditions such that the mechanical strength of sepiolite can be enhanced. Specifically, preliminary firing at about 400°C to about 800°C is considered to be preferred.

### <Test Example 8>

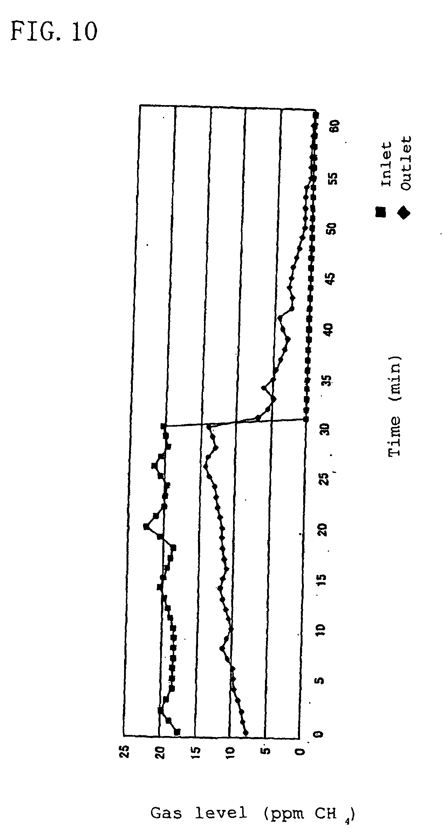

Test Example 8 was carried out in a manner similar to that of Test Example 2 employing the deodorizing article of Example 2. After the adsorption step had been performed for 31 minutes, the outlet gas concentration was determined at every one minute while desorption was performed. The results are shown in Fig. 10.

The results indicate that the odor substance adsorbed over approximately 30 minutes was nearly completely released for 60 minutes.

### <Test Example 9>

The deodorizing article 10 shown in Fig. 1 (composition: that employed in Example 2) was subjected to the deodorization test in the following manner.

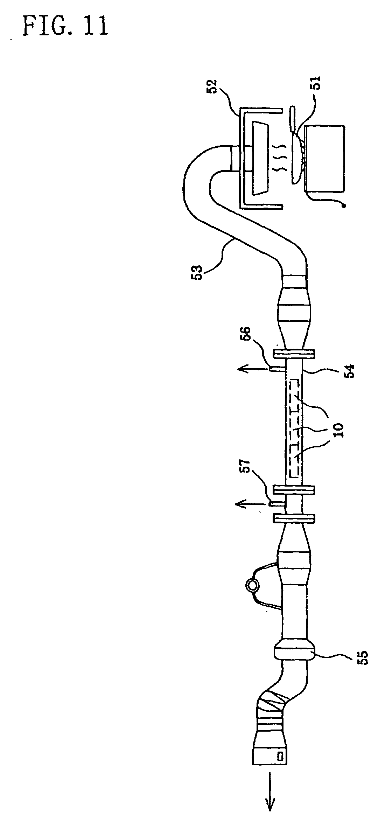

As shown in Fig. 11, a duct 53 equipped with a hood 52 was provided above a frying pan 51 such that the hood faces the frying pan. The end of the duct 53 opposite the hood side was connected to a sample-charged duct 54 in which three deodorizing articles 10 are arranged in line. A fan 55 was provided in a line disposed on the downstream side with respect to the sample-charged duct 54, so as to discharge the gas.

Salad oil (200 mL) was added to the frying pan 51 and heated at 200°C so as to generate oil-related gas. The gas was caused to pass through the duct for five minutes at a deodorizing-article-passage speed of 4 m/s (deodorizing-article-flow rate of 1.5 m³/min). The gas was sampled simultaneously at an inlet sampling position 56 and an outlet sampling position 57 of the sample-charged duct 54, thereby determining the gas concentration through the odor concentration method and calculating the percent removal of odor.

After oil-related gas had been caused to flow for 5 minutes, the frying pan 51 was removed, and odorless air was caused to flow for 20 minutes for idling operation.

Thereafter, the sample-charged duct 54 was removed and allowed to stand under the conditions of 32°C and 50% RH for two days. The procedure was repeated.

The results are shown in Table 1. The total surface area of the deodorizing article 10 was found to be 676 cm².

Specifically, the above determination on the basis of the odor concentration method was performed through the triangle bag method for odor sensory measurement, in accordance with "Method for Calculating Odor Index" instructed by the bulletin No. 63 of the Environment Agency, Japan. As shown in Table 2, the panel consists of six standard panelists. The maximum score and minimum score were discarded, and the remaining scores provided by four panelists were averaged, to thereby obtain the odor level.

**Table 1**

| | Passage flow rate (Passage speed) | Temp. & humidity conditions | Odor concentration | | Percent removal |
|---|---|---|---|---|---|
| | | | Inlet | Outlet | |
| Initial | 1.5 m³/min (4.0 m/s) | 29°C, 46% RH | 730 | 170 | 77% |
| First repetition | | 30°C, 50% RH | 730 | 170 | 77% |

**Table 2**

| | Test Example 9 | | Test Example 10 | |
|---|---|---|---|---|
| | Initial | First repetition | Initial | After desorption |
| A: Female in 20s | O | O | O | O |
| B: Female in 20s | O | O | O | O |
| C: Male in 30s | O | O | O | O |
| D: Male in 40s | O | X | X | X |
| E: Male in 30s | O | O | O | O |
| F: Male in 30s | O | O | O | O |
| G: Male in 30s | X | O | X | X |
| H: Male in 30s | X | X | O | O |
| O: Panelist participating in the respective test | | | | |

As shown in the Tables, a percent removal of odor of 77% can be attained when the odorous gas is treated. The outlet odor concentration does not exceed the regulation standard by the Tokyo Metropolitan Environmental Ordinance. Thus, repeated use is considered to cause no substantial deterioration in deodorization performance.

### <Test Example 10>

In a manner similar to that employed in Test Example 9, the deodorizing article 10 shown in Fig. 1 (composition: that employed in Example 2) was subjected to the deodorization test in the following manner.

A duct 53 equipped with a hood 52 was provided above a frying pan 51 such that the hood faces the frying pan. The end of the duct 53 opposite the hood side was connected to a sample-charged duct 54 in which three deodorizing articles 10 are placed. A fan 55 was provided in a line disposed on the downstream side with respect to the sample-charged duct 54, so as to discharge the gas.

Salad oil (200 mL) was added to the frying pan 51 and heated at 200°C so as to generate oil-related gas. The gas was caused to pass through the duct for five minutes at a deodorizing-article-passage speed of 4 m/s (deodorizing-article-flow rate of 1.5 m³/min). The gas was sampled simultaneously at an inlet sampling position 56 and an outlet sampling position 57 of the sample-charged duct 54, thereby determining the gas concentration through the odor concentration method and calculating the percent removal of odor.

After oil-related gas had been caused to flow for 5 minutes, the frying pan 51 was removed, and odor-less air was caused to flow for 20 minutes for idling operation. Immediately after the termination of idling operation, the gas was sampled simultaneously at an inlet sampling position 56 and an outlet sampling position 57, thereby determining the gas concentration through the odor concentration method.

The results are shown in Table 3. The panelists who were involved in the determination through the odor concentration method are shown in Table 2.

**Table 3**

| | Passage flow rate (Passage speed) | Temp. & humidity conditions | Odor concentration | | Percent removal |
|---|---|---|---|---|---|
| | | | Inlet | Outlet | |
| Initial | 1.5 m³/min (4.0 m/s) | 29°C, 50% RH | 980 | 230 | 77% |
| After desorption | | 30°C, 47% RH | (≤10) | 17 | ― |

As shown in Table 3, the percent removal of odor reaches as high as that of 77% when the odorous gas is treated. In addition, when the odorous gas does not flow, the deodorizing article is confirmed to release the adsorbed odor. Thus, the results sustain that the deodorizing article can be used repeatedly and semi-permanently. The outlet odor level during desorption is confirmed to be remarkably lower than the regulation standard in accordance with the Tokyo Metropolitan Environmental Ordinance.

As described above, the deodorizing article which is placed in an exhaust system connected to the kitchens or similar facilities can remove odor at high efficiency when the odorous gas is treated, and gradually releases the adsorbed odor through desorption when the gas contains few odor. Thus, the deodorizing article of the present invention is suitable used in a mode in which the outlet odor level is maintained at a level equal to or lower than the regulation standard semi-permanently in a maintenance free-manner.

### Industrial Applicability

As described hereinabove, by virtue of synergistic effect exerted by gypsum series and a clay mineral having a tunnel-like micro-structure such as sepiolite or synergistic effect exerted through addition of a mineral having macropores of a pore size greater than that of the gypsum series (e.g., perlite), the deodorizing article of the present invention adsorbs an odor substance to thereby lower the odor level to a certain level or lower, when the target gas contains a large amount of an odor component, and releases the odor component without elevating the level of the released odor component to a certain level or higher, when the odor component level is lowered. By repeating the procedure, the odor level can be averaged and lowered to a certain level or lower, with the level being suppressed semi-permanently.

## Claims

1. A deodorizing article for use in an exhaust system connected to a kitchen, particularly for removing an oil-related odor generated in the kitchen, said deodorizing article comprising:
at least 25 wt.% of gypsum series with macro-pores having a pore size of 1 to 10 µm; and
at least 30 wt.% of a clay mineral having a tunnel-like micro-structure and dispersed in the gypsum series, which clay mineral is selected from the group consisting of sepiolite and palygorskite,
wherein the deodorizing article adsorbs the odor component thereto and releases the adsorbed odor component when the odor component concentration is lowered.

2. The article of claim 1, wherein the adsorption effect is renewed through enzymatic reaction involved with a microorganism adhering thereon.

3. The article of claim 1 or 2, which has a hollow cylindrical or columnar form and has a flow line penetrated in an axial direction.

4. The article of any preceding claim, which comprises in addition to the clay mineral a mineral having a pore size greater than that of the gypsum series.

5. The article of claim 4, wherein the additional mineral is expanded perlite.

6. The article of claim 4 or 5, which contains the additional mineral in an amount of 30 wt.% or less.

7. A method for producing the deodorizing article of claim 1, comprising the steps of:
subjecting the clay mineral to preliminary firing at a predetermined temperature;
dispersing the thus-fired clay mineral in the gypsum series and charging the resultant mixture in a mold; and
drying the mixture and releasing the dried product from the mold.

8. The method of claims 7, further comprising the step of adding to then clay mineral a mineral having a pore size greater than that of the gypsum series.

9. The method of claim 8, wherein the additional mineral is expanded perlite.

10. The method of claim 8 or 9, wherein the additional mineral is added in an amount of 30 wt.% or less.

## Patentansprüche

1. Desodorierender Artikel zur Verwendung in einem an eine Küche angeschlossenen Abzugssystem, insbesondere zum Entfernen eines in der Küche erzeugten öligen Geruchs, wobei der desodorierende Artikel aufweist:
wenigstens 25 Gew.-% eines Gipsmaterials mit Makroporen, die eine Porengröße von 1 bis 10 µm aufweisen; und
wenigstens 30 Gew.-% eines in dem Gipsmaterial dispergierten Tonminerals mit einer tunnelartigen Mikrostruktur, wobei das Tonmineral Sepiolith oder Palygorskit ist,
wobei der desodorierende Artikel die Geruchskomponente adsorbiert und die adsorbierte Geruchskomponente abgibt, wenn sich die Geruchskomponentenkonzentration erniedrigt.

2. Artikel nach Anspruch 1, wobei sich die Adsorptionswirkung durch eine enzymatische Reaktion erneuert, an der ein daran anhaftender Mikroorganismus beteiligt ist.

3. Artikel nach Anspruch 1 oder 2, der eine hohle zylindrische oder säulenartige Form und einen in Axialrichtung durchdringenden Strömungsverlauf aufweist.

4. Artikel nach einem der vorstehenden Ansprüche, der zusätzlich zu dem Tonmineral ein Mineral mit einer größeren Porengröße als das Gipsmaterial aufweist.

5. Artikel nach Anspruch 4, wobei das zusätzliche Mineral expandiertes Perlit ist.

6. Artikel nach Anspruch 4 oder 5, der das zusätzliche Mineral in einem Betrag von höchstens 30 Gew.-% aufweist.

7. Verfahren zum Herstellen des desodorierenden Artikels nach Anspruch 1, wobei:
das Tonmineral einem vorbereitenden Brennen bei einer vorbestimmten Temperatur unterzogen wird;
das gebrannte Tonmineral in dem Gipsmaterial dispergiert und die entstandene Mischung in eine Form gegeben wird; und
die Mischung getrocknet und das getrocknete Produkt aus der Form entfernt wird.

8. Verfahren nach Anspruch 7, wobei ferner zu dem Tonmineral ein Mineral mit einer Porengröße zugegeben wird, die größer ist als die des Gipsmaterials.

9. Verfahren nach Anspruch 8, wobei das zusätzliche Mineral expandiertes Perlit ist.

10. Verfahren nach Anspruch 8 oder 9, wobei das zusätzliche Mineral in einem Betrag von höchstens 30 Gew.-% zugegeben wird.

## Revendications

1. Article désodorisant destiné à être utilisé dans un système de ventilation relié à une cuisine, en particulier pour enlever une odeur liée à l'huile générée dans la cuisine, ledit article désodorisant comprenant :
au moins 25 % en poids d'une série du gypse avec des macropores ayant une grosseur de pores de 1 à 10 µm ; et
au moins 30 % en poids d'un minéral argileux ayant une microstructure de type tunnel et étant dispersé dans la série du gypse, lequel minéral argileux est choisi dans le groupe composé de sépiolite et de palygorskite,
l'article désodorisant adsorbant le composant de l'odeur et libérant le composant de l'odeur adsorbé lorsque la concentration en composant de l'odeur est abaissée.

2. Article selon la revendication 1, dans lequel l'effet d'adsorption est renouvelé par une réaction enzymatique qui implique un micro-organisme adhérant à celui-ci.

3. Article selon la revendication 1 ou 2, lequel a une forme cylindrique ou colonnaire creuse et qui a une ligne d'écoulement pénétrée dans une direction axiale.

4. Article selon l'une quelconque des revendications précédentes, lequel comprend en plus du minéral argileux un minéral ayant une grosseur de pores supérieure à celle de la série du gypse.

5. Article selon la revendication 4, dans lequel le minéral supplémentaire est de la perlite expansée.

6. Article selon la revendication 4 ou 5, lequel contient le minéral supplémentaire en quantité de 30 % en poids ou moins.

7. Procédé de production de l'article désodorisant selon la revendication 1, comprenant les étapes consistant à:
soumettre le minéral argileux à une cuisson préliminaire à une température prédéterminée ;
disperser le minéral argileux ainsi cuit dans la série du gypse et à charger le mélange obtenu dans un moule ; et
sécher le mélange et libérer le produit séché du moule.

8. Procédé selon la revendication 7, comprenant en outre l'étape consistant à ajouter au minéral argileux un minéral ayant une grosseur de pores supérieure à celle de la série du gypse.

9. Procédé selon la revendication 8, dans lequel le minéral supplémentaire est de la perlite expansée.

10. Procédé selon la revendication 8 ou 9, dans lequel le minéral supplémentaire est ajouté en quantité de 30 % en poids ou moins.
